(19) Europäisches Patentamt
European Patent Office
Office européen des brevets

(11) Veröffentlichungsnummer : **0 364 538 B1**

(12) # EUROPÄISCHE PATENTSCHRIFT

(45) Veröffentlichungstag der Patentschrift : **06.05.92 Patentblatt 92/19**

(21) Anmeldenummer : **89903715.4**

(22) Anmeldetag : **28.02.89**

(86) Internationale Anmeldenummer : **PCT/EP89/00188**

(87) Internationale Veröffentlichungsnummer : **WO 89/08633 21.09.89 Gazette 89/23**

(51) Int. Cl.[5] : **C07C 43/225,** C09K 19/30, C07C 69/75, C07C 69/92, C07C 69/24, C07C 69/76, C07C 25/18

(54) **DIFLUORBENZOLDERIVATE.**

(30) Priorität : **10.03.88 DE 3807872**

(43) Veröffentlichungstag der Anmeldung : **25.04.90 Patentblatt 90/17**

(45) Bekanntmachung des Hinweises auf die Patenterteilung : **06.05.92 Patentblatt 92/19**

(84) Benannte Vertragsstaaten : **CH DE FR GB IT LI NL**

(56) Entgegenhaltungen :
**EP-A- 0 117 631**
**EP-A- 0 125 563**
**EP-A- 0 133 489**

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **REIFFENRATH, Volker**
**Jahnstrasse 18**
**W-6101 Rossdorf (DE)**
Erfinder : **KRAUSE, Joachim**
**Samuel-Morse-Str. 14**
**W-6110 Dieburg (DE)**
Erfinder : **WÄCHTLER, Andreas**
**Goethestr. 34**
**W-6103 Griesheim (DE)**
Erfinder : **WEBER, Georg**
**Wilhelm-Leuschner-Str. 38**
**W-6106 Erzhausen (DE)**
Erfinder : **FINKENZELLER, Ulrich**
**Waldpfad 74**
**W-6831 Plankstadt (DE)**

**EP 0 364 538 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Difluorbenzolderivate der Formel I,

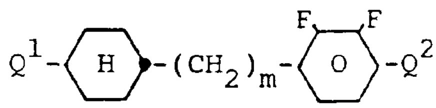

I

worin

$Q^1$ und $Q^2$ jeweils unabhängig voneinander R oder R-$A^1$-$A^2$-Z bedeuten, wobei

R jeweils unsubstituiertes, einfach durch Cyan oder mindestens einfach durch Fluor oder Chlor substituiertes Alkyl mit 1-15 C-Atomen oder Alkenyl mit 3-15 C-Atomen, wobei in diesen Resten auch eine $CH_2$-Gruppe durch -O-, -CO-, -O-CO-, -CO-O- oder -O-CO-O- ersetzt sein kann,

$A^1$ und $A^2$ jeweils unabhängig voneinander trans-1,4-Cyclohexylen, worin auch eine oder zwei nicht benachbarte $CH_2$-Gruppen durch -O- ersetzt sein können, oder unsubstituiertes oder ein oder zweifach durch Fluor substituiertes 1,4-phenylen, worin auch eine oder zwei CH-Gruppen durch N ersetzt sein können; eine der beiden Gruppen $A^1$ und $A^2$ auch eine Einfachbindung,

Z -CO-O-, -O-CO-, $-CH_2O-$, $-OCH_2-$, $-CH_2CH_2-$ oder eine Einfachbindung bedeutet, und

m 0 oder 2 ist,

mit der Maßgabe, daß im Falle

(a)

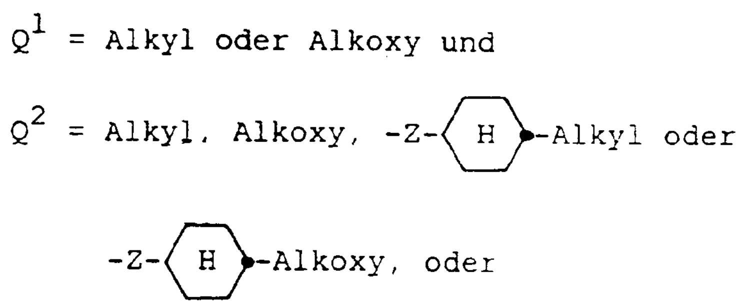

(b)

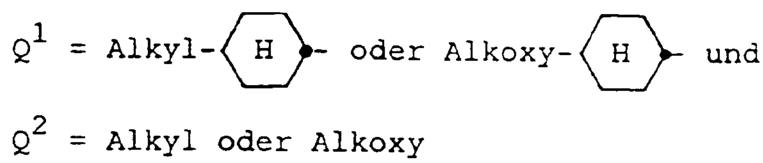

m = 0 bedeutet.

Der Einfachheit halber bedeuten im folgenden Cyc eine 1,4-Cyclohexylengruppe, Phe eine 1,4-Phenylengruppe, Pyd eine Pyridin-2,5-diylgruppe, Pyr eine Pyrimidin-2,5-diylgruppe, Pyn eine Pyrazin-2,5-diylgruppe, wobei diese Gruppen unsubstituiert oder durch ein oder zwei Fluor substituiert sein können. Vorzugsweise sind diese Gruppen unsubstituiert.

$PheF_2$ bedeutet eine Gruppe der Formel

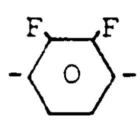

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Die Verbindungen der Formel I zeichnen sich durch eine deutlich negative Anisotropie der Dielektrizitätskonstante aus und werden in einem elektrischen Feld mit ihren Moleküllängsachsen senkrecht zur Feldrichtung ausgerichtet. Dieser Effekt ist bekannt und wird zur Steuerung der optischen Transparenz in verschiedenen Flüssigkristallanzeigen ausgenützt, so z.B. in Flüssigkristallzellen vom Lichtstreuungstyp (dynamische Streuung), vom sogenannten DAP-Typ (Deformation aufgerichteter Phasen) oder ECB-Typ (electrically controlled birefringence) oder vom Gast/Wirt-Typ (guest host interaction).

Weiterhin eignen sich Verbindungen der Formel I als Komponenten chiral getilteter smektischer Phasen. Chiral getiltete smektische flüssigkristalline Phasen mit ferroelektrischen Eigenschaften können hergestellt werden, in dem man Basis-Mischungen mit einer oder mehreren getilteten smektischen Phasen mit einem geeigneten chiralen Dotierstoff versetzt (L.A. Veresnev et al., Mol. Cryst. Liq. Cryst. 89, 327 (1982); H.R. Brand et al., J. Physique 44 (lett.), L-771 (1983)).Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen.

Es sind bisher bereits eine Reihe von flüssigkristallinen Verbindungen mit schwach negativer dielektrischer Anisotropie synthetisiert worden. Hingegen sind noch relativ wenig Flüssigkristallkomponenten mit großer negativer Anisotropie der Dielektrizitätskonstante bekannt. Zudem weisen letztere im allgemeinen Nachteile auf, wie z.B. schlechte Löslichkeit in Mischungen, hohe Viskosität, hohe Schmelzpunkte und chemische Instabilität. Es besteht daher ein Bedarf an weiteren Verbindungen mit negativer dielektrischer Anisotropie, die es erlauben, die Eigenschaften von Mischungen für verschiedenste elektro-optische Anwendungen weiter zu verbessern.

Flüssigkristallverbindungen mit negativer dielektrischer Anisotropie, welche zwei oder drei über Carboxylgruppen oder Kovalenzbindung verknüpfte Ringe und eine oder mehrere seitliche Gruppen, wie Halogen, Cyano oder Nitrogruppen aufweisen, sind bekannt aus DE 22 40 864, DE 26 13 293, DE 28 35 662, DE 28 36 086 und EP 023 728.

In der EP 084 194 werden in einer breiten Formel die hier beanspruchten Verbindungen umfaßt. Jedoch sind dort keine Einzelverbindungen der erfindungsgemäßen Formel genannt. Der Fachmann konnte somit aus dem Stand der Technik weder in einfacher Art und Weise Synthesemöglichkeiten für die beanspruchten Verbindungen entnehmen noch erkennen, daß die erfindungsgemäßen Verbindungen überwiegend günstig gelegene Mesophasenbereiche aufweisen und sich durch eine große negative Anisotropie der Dielektrizität bei gleichzeitiger niedriger Viskosität auszeichnen.

Auch fehlt dort jeglicher Hinweis auf die Möglichkeit des Einsatzes der erfindungsgemäßen Verbindungen in Displays, die auf der SSFLC-Technologie beruhen, da die dort beanspruchten Verbindungen geringe smektische Tendenzen aufweisen.

Weiterhin sind Dibenzoesäureester des 2,3-Dichlorhydrochinons bekannt (z.B. Bristol et al., J. Org. Chem. 39, 3138 (1974) oder Clanderman et al., J. Am. Chem Soc 97, 1585 (1975)), die allerdings monotrop sind bzw. sehr kleine Mesophasenbereiche aufweisen. Die von Eidenschink et al. beschriebenen Ester der 4-Hydroxy-2,3-dichlorbenzoesäure (Angew. Chem. 89, 103 (1977)) besitzen ebenfalls nur schmale Mesophasenbereiche.

Die aus der DE OS 29 33 563 bekannten 4-Alkyl-2,3-dichlorphenyl-4′-alkylbicyclohexyl-4-carbonsäureester erlauben wegen ihrer hohen Viskosität keine technische Anwendung.

Der Erfindung lag die Aufgabe zugrunde stabile, flüssigkristalline oder mesogene Verbindungen mit einer großen negativen Anisotropie der Dielektrizität und gleichzeitig geringer Viskosität aufzuzeigen.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit breitem Mesophasenbereich und vergleichsweise niedriger Viskosität herstellbar.

Weiterhin eignen sich die Verbindungen der Formel I als Komponenten chiral getilteter smektischer flüssigkristalliner Phasen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder die Phasenbereiche und/oder den Tiltwinkel und/oder den Pitch eines solchen Dielektrikums zu varriieren.

Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine entsprechende Carbonsäure oder eines ihrer raktionsfähigen Derivate mit einer entsprechenden Hydroxyverbindung oder einem seiner reaktionsfähigen Derivate umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I, sowie Flüssigkristall-Anzeigeelemente, die derartige Phasen enthalten. Derartige phasen weisen besonders vorteilhafte elastische Konstanten auf, und eignen sich wegen ihrer niedrigen $\Delta\varepsilon/\varepsilon_\perp$, -Werte besonders für TFT-Mischungen.

Vor- und nachstehen haben $Q^1$, $Q^2$, $A^1$, $A^2$ , R, Z und m die angegebene Bedeutung, sofern nicht ausdrücklich etwas anderes vermerkt ist.

Die Verbindungen der Formel I umfassen dementsprechend bevorzugte Verbindungen der Teilformeln Ia und Ib (mit zwei Ringen), Teilformeln Ic bis If (mit drei Ringen) und Ig bis Im (mit vier Ringen):

| | |
|---|---|
| R-Cyc-$PheF_2$-R | Ia |
| R-Cyc-$CH_2CH_2$-$PheF_2$-R | Ib |
| R-$A^1$-Z-Cyc-$PheF_2$-R | Ic |
| R-$A^1$-Z-Cyc-$CH_2CH_2$-$PheF_2$-R | Id |
| R-Cyc-$PheF_2$-Z-$A^1$-R | Ie |
| R-Cyc-$CH_2CH_2$-$PheF_2$-Z-$A^1$-R | If |
| R-$A^1$-Z-Cyc-$PheF_2$-Z-$A^1$-R | Ig |
| R-$A^1$-Z-Cyc-$CH_2CH_2$-$PheF_2$-Z-$A^1$-R | Ih |
| R-$A^1$-$A^2$-Z-Cyc-$PheF_2$-R | Ii |
| R-$A^1$-$A^2$-Z-Cyc-$CH_2CH_2$-$PheF_2$-R | Ik |
| R-Cyc-$PheF_2$-Z-$A^2$-$A^1$-R | Il |
| R-Cyc-$CH_2CH_2$-$PheF_2$-Z-$A^2$-$A^1$-R | Im |

Darunter sind diejenigen der Teilformeln Ia bis If besonders bevorzugt.

In den Verbindungen der vor- und nachstehenden Formeln bedeutet R jeweils unabhängig voneinander vorzugsweise Alkyl oder Alkoxy.

Weiterhin bevorzugt sind Verbindungen der vor- und nachstehenden Formeln, in denen einer der Reste R Alkenyl oder Oxaalkyl (z.B. Alkoxymethyl) bedeutet.

$A^1$ und $A^2$ sind bevorzugt unsubstituiertes 1,4-Phenylen, Cyc, Pyd oder Pyr; bevorzugt enthält die Verbindung der Formel I nicht mehr als jeweils einen der Reste Pyd, Pyn, Pyr oder 2,3-Difluor-1,4-phenylen.

Die Reste R weisen in den vor- und nachstehenden Formeln vorzugsweise 2-12 C-Atome, insbesondere 3-10 C-Atome auf. In R können auch eine oder zwei $CH_2$-Gruppen ersetzt sein. Vorzugsweise ist nur eine $CH_2$-Gruppe ersetzt durch -O-, oder -CH=CH-.

In den vor- und nachstehenden Formeln bedeuten die Reste R vorzugsweise Alkyl, Alkoxy oder eine andere Oxaalkylgruppe, ferner auch Alkylgruppen, in denen eine oder zwei $CH_2$-Gruppen durch -CH=CH- ersetzt sein können.

Falls die Reste R Alkylreste, in denen auch eine ("Alkoxy" bzw. "Oxaalkyl") oder zwei ("Alkoxyalkoxy" bzw. "Dioxaalkyl") nicht benachbarte $CH_2$-Gruppen durch O-Atome ersetzt sein können, bedeuten, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxypropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4-, 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Falls die Reste R einen Alkylrest bedeuten, in dem eine $CH_2$-Gruppe durch -CH = CH- ersetzt ist, so ist die transForm bevorzugt. Dieser Alkenylrest kann geradkettig oder verzweigt sein. Vorzugsweise ist er geradkettig und hat 2 bis 10 C-Atome Er bedeutet demnach besonders Vinyl, Prop-1-, oder Prop-2-enyl, But-1-, 2- oder But-3-enyl, Pent-1- 2-, 3- oder Pent-4-enyl, Hex-1-, 2-, 3-, 4- oder Hex-5-enyl, Hept-1-, 2-, 3-, 4-, 5- oder Hept-6-enyl Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formel I mit verzweigten Resten R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy.

Formel I umfaßt sowohl die Racemate dieser Verbindungen als auch die optischen Antipoden sowie deren Gemische.

Unter den Verbindungen der Formeln I sowie Ia bis Ik sind diejenigen bevorzugt, in denen mindestens einer der darin enthaltenen Reste eine der angegebenen bevorzugten Bedeutungen hat.

Die Verbindungen der Formel I werden nach an sich bekannten Methoden hergestellt, wie sie in der Literatur (z. B in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I sind ausgehend von 1,2-Difluorbenzol zugänglich. Dieses wird nach bekanntem Verfahren (z.B. A.M. Roe et al, J. Chem. Soc. Chem. Comm., 22, 582 (1965)) metalliert und mit dem entsprechenden Elektrophil umgesetzt. Mit dem so erhaltenen 1-substituierten 2,3-Difluorbenzol läßt sich diese Reaktionssequenz ein zweites Mal mit einem geeigneten Elektrophil durchführen und man gelangt so zu 1,4-disubstituierte 2,3-Difluorbenzolen, die ggf. in weiteren Reaktionsschritten in die Endprodukte überführt werden können. 1,2-Difluorbenzol bzw. 1-substituiertes 2,3-Difluorbenzol wird in einem inerten Lösungsmittel wie Diethylether, Tetrahydrofuran, Dimethoxyethan, tert-Butylmethylether oder Dioxan, Kohlenwasserstoffen wie Hexan, Heptan, Cyclohexan, Benzol oder Toluol oder Gemischen dieser Lösungsmittel gegebenenfalls unter Zusatz eine Komplexierungsmittels wie Tetramethylethylendiamin (TMEDA) oder Hexamethylphosphorsäuretriamid mit Phenyllithium, Lithiumtetramethylpiperidin, n-, sek- oder tert-Butyllithium bei Temperaturen von -100 °C bis +50 °C vorzugsweise -78 °C bis 0 °C umgesetzt.

Die Lithium-2,3-difluorphenyl-Verbindungen werden bei -100 °C bis 0 °C vorzugsweise unter -50 °C mit den entsprechenden Elektrophilen umgesetzt. Geeignete Elektrophile sind Aldehyde Ketone, Nitrile, Epoxide, Carbonsäure-Derivate wie Ester, Anhydride oder Halogenide Halogenameisensäureester oder Kohlendioxid.

Zur Umsetzung mit aliphatischen oder aromatischen Halogen-Verbindungen werden die Lithium-2,3-difluorphenyl-Verbindungen transmetalliert und unter Übergangsmetallkatalyse gekoppelt. Besonders geeignet sind hierfür die Zink- (vgl. DE OS 36 32 410) oder die Titan-2,3-difluorphenyl-Verbindungen (vgl. DE OS 37 36 489).

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer $-CH_2CH_2-$Gruppe eine -CH=CH-Gruppe und/oder an Stelle einer $-CH_2-$Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen 0° und etwa 200 ° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Cagliotti [durch Umsetzung der Tosylhydrazone mit Natriumborhydrid, Natriumcyanborhydrid oder Catecholboran (Org. Synth. 52, 122 (1972) ] zu den entsprechenden

Verbindungen der Formel I, die Alkylgruppen und/oder $-CH_2CH_2$-Brücken enthalten, reduziert werden.

Weiterhin sind Reduktionen mit komplexen Hydriden möglich beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°.

Die erfindungsgemäßen Ester können
durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Dyethylether, Di-n-butylether, THF, Dioxan oder Anisol, Ketone wie Aceton, Butanon oder Cyclohexanon, Amide wie DMF oder Phosphorsäure-hexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 ° und +250 °, vorzugsweise zwischen -20 ° und +80 °. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Collidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25 ° und +20 °.

Erfindungsgemäße Ether sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2 -Dimethoxyethan, DMF oder Dimethylsulfoxid oder auch einem Überschuß an wäßriger oder wäßrig-alkoholischer NaOH oder KOH bei Temperaturen zwischen etwa 20° und 100 °C.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4′-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R′-L-E-R″ 1
R′-L-COO-E-R″ 2
R′-L-OOC-E-R″ 3
R′-L-$CH_2CH_2$-E-R″ 4
R′-L-C≡C-E-R″ 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R′ und R″ bedeuten in den Verbindungen der Teilformeln 1a, 2a, 3a, 4a und 5a jeweils unabhängig voneinander Alkyl Alkenyl, Alkoxy, Alkenyloxy oder Alkanoyloxy mit bis zu 8 Kohlenstoffatomen. Bei den meisten dieser Verbindungen sind R′ und R″ voneinander verschieden, wobei einer dieser Reste meist Alkyl oder Alkenyl ist. In den Verbindungen der Teilformeln 1b 2b, 3b, 4b und 5b bedeutet R″ -CN, -$CF_3$, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,
Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

39 g 1-(4-Ethoxy-2,3-difluorphenyl)-4-(4-trans-propylcyclohexylethyl)cyclohex-1-en (Herstellung: Unter Feuchtigkeitsausschluß und Stickstoffatmosphäre werden zu einer Lösung von 31,6 g Ethoxy-2,3-difluorbenzol und 23,2 g Tetramethylethylendiamin (TMEDA) in 400 ml Tetrahydrofuran (THF) bei -70 °C 131 ml einer 1,6 n Lösung von Butyllithium (BuLi) in n-Hexan getropft. Anschließend rührt man 4 Stunden bei -70 °C und gibt dann langsam 50 g 4-(trans-4-n-Propylcyclohexylethyl)-cyclohexanon in 100 ml THF zu. Man läßt das Reaktionsgemisch langsam auf Raumtemperatur erwärmen und hydrolysiert dann mit 1,5 l einer gesättigten Ammoniumchloridlösung. Das Gemisch wird mit Ether extrahiert und die Etherphase mehrmals mit Wasser gewaschen, getrocknet und eingedampft. Der Rückstand wird in 700 ml Ethanol aufgenommen, mit 70 ml konz. HCl versetzt und 3 Stunden am Rückfluß gekocht. Anschließend gibt man 1,5 l Wasser hinzu, extrahiert mit Methyltertiärbutylether (MTB-Ether). Die Etherphase wird neutral gewaschen, getrocknet, eingedampft und der Rückstand umkristallisiert) werden in 250 ml THF gelöst und in Gegenwart von 4 g Pd-C-5 % bei 34 °C unter 0,5 bar Wasserstoffdruck hydriert. Anschließend wird filtriert, die Lösung eingedampft und chromatographisch und durch Kristallisation trans-4-(trans-4-n-Propylcyclohexylethyl)-(4-ethoxy-2,3-difluorphenyl)-cyclohexan isoliert.

Analog werden hergestellt:

trans-4-(4-Ethylcyclohexylethyl)-(4-ethoxy-2,3-difluorphenyl)-cyclohexan
trans-4-(4-Butylcyclohexylethyl)-(4-ethoxy-2,3-difluorphenyl)-cyclohexan
trans-4-(4-Pentylcyclohexylethyl)-(4-ethoxy-2,3-difluorphenyl)-cyclohexan
trans-4-(4-Heptylcyclohexylethyl)-(4-ethoxy-2,3-difluorphenyl)-cyclohexan
trans-4-(4-Ethylcyclohexylethyl)-(4-propoxy-2,3-difluorphenyl)-cyclohexan
trans-4-(4-Propylcyclohexylethyl)-(4-propoxy-2,3-difluorphenyl)-cyclohexan
trans-4-(4-Butylcyclohexylethyl)-(4-propoxy-2,3-difluorphenyl)-cyclohexan
trans-4-(4-Pentylcyclohexylethyl)-(4-propoxy-2,3-difluorphenyl)-cyclohexan
trans-4-(4-Heptylcyclohexylethyl)-(4-propoxy-2,3-difluorphenyl)-cyclohexan
trans-4-(4-Ethylcyclohexylethyl)-(4-butoxy-2,3-difluorphenyl)-cyclohexan
trans-4-(4-Propylcyclohexylethyl)-(4-butoxy-2,3-difluorphenyl)-cyclohexan
trans-4-(4-Butylcyclohexylethyl)-(4-butoxy-2,3-difluorphenyl)-cyclohexan
trans-4-(4-Pentylcyclohexylethyl)-(4-butoxy -2,3-difluorphenyl)-cyclohexan
trans-4-(4-Heptylcyclohexylethyl)-(4-butoxy-2,3-difluorphenyl)-cyclohexan
trans-4-(4-Ethylcyclohexylethyl)-(4-pentyloxy-2,3-difluorphenyl)-cyclohexan
trans-4-(4-Propylcyclohexylethyl)-(4-pentyloxy-2,3-difluorphenyl)-cyclohexan
trans-4-(4-Butylcyclohexylethyl)-(4-pentyloxy-2,3-difluorphenyl)-cyclohexan
trans-4-(4-Pentylcyclohexylethyl)-(4-pentyloxy-2,3-difluorphenyl)-cyclohexan
trans-4-(4-Heptylcyclohexylethyl)-(4-pentyloxy-2,3-difluorphenyl)-cyclohexan
trans-4-(4-Ethylcyclohexylethyl)-(4-heptyloxy-2,3-difluorphenyl)-cyclohexan
trans-4-(4-Propylcyclohexylethyl)-(4-heptyloxy-2,3-difluorphenyl)-cyclohexan
trans-4-(4-Butylcyclohexylethyl)-(4-heptyloxy-2,3-difluorphenyl)-cyclohexan
trans-4-(4-Pentylcyclohexylethyl)-(4-heptyloxy-2,3-difluorphenyl)-cyclohexan
trans-4-(4-Heptylcyclohexylethyl)-(4-heptyloxy-2,3-difluorphenyl)-cyclohexan

Beispiel 2

Zu einer gut gerührten Lösung von 12,3 g Kaliumtertiärbutylat (t-BuOK) in 80 ml THF gibt man nacheinander bei -90 °C eine Lösung von 37,5 g 1,2-Difluor-3-(trans-4-n-propylcyclohexylmethoxy)-benzol in 70 ml THF, 68,5 ml einer 1,6 n Lösung von Butyllithium (BuLi) in Hexan und dann eine Lösung von 39,8 g trans-4-(trans-4-n-Propylcyclohexyl)cyclohexylethyljodid und 20 g 1,3-Dimethyltetrahydro-2-(1H)-pyrimidinon (DMPU) in 70 ml THF. Nachdem die Zugabe beendet ist läßt man die schlecht rührbare Suspension langsam auf -40 °C erwärmen, gibt Wasser zu und arbeitet wie üblich auf. Man erhält trans-4-propylcyclohexylmethyl-[4-(trans-4-(trans-4-propylcyclohexyl)-cyclohexylethyl)-2,3-difluorphenyl]-ether.

Analog werden hergestellt:

trans-4-Ethylcyclohexylmethyl-[4-(trans-4-(trans-4-propylcyclohexyl)-cyclohexylethyl)-2,3-difluorphenyl]-ether
trans-4-Butylcyclohexylmethyl-[4-(trans-4-(trans-4-propylcyclohexyl)-cyclohexylethyl)-2,3-difluorphenyl]-ether
trans-4-Pentylcyclohexylmethyl-[4-(trans-4-(trans-4-propylcyclohexyl)-cyclohexylethyl)-2,3-difluorphenyl]-ether
trans-4-Ethylcyclohexylmethyl-[4-(trans-4-(trans-4-butylcyclohexyl)-cyclohexylethyl)-2,3-difluorphenyl]-ether
trans-4-Propylcyclohexylmethyl-[4-(trans-4-(trans-4-butylcyclohexyl)-cyclohexylethyl)-2,3-difluorphenyl]-ether
trans-4-Butylcyclohexylmethyl-[4-(trans-4-(trans-4-butylcyclohexyl)-cyclohexylethyl)-2,3-difluorphenyl]-ether
trans-4-Heptylcyclohexylmethyl-[4-(trans-4-(trans-4-butylcyclohexyl)-cyclohexylethyl)-2,3-difluorphenyl]-ether
trans-4-Ethylcyclohexylmethyl-[4-(trans-4-(trans-4-pentylcyclohexyl)-cyclohexylethyl)-2,3-difluorphenyl]-ether
trans-4-Propylcyclohexylmethyl-[4-(trans-4-(trans-4-pentylcyclohexyl)-cyclohexylethyl)-2,3-difluorphenyl]-ether
trans-4-Butylcyclohexylmethyl-[4-(trans-4-(trans-4-pentylcyclohexyl)-cyclohexylethyl)-2,3-difluorphenyl]-ether
trans-4-Pentylcyclohexylmethyl-[4-(trans-4-(trans-4-pentylcyclohexyl)-cyclohexylethyl)-2,3-difluorphenyl]-ether
trans-4-Ethylcyclohexylmethyl-[4-(trans-4-(trans-4-heptylcyclohexyl)-cyclohexylethyl)-2,3-difluorphenyl]-ether
trans-4-Propylcyclohexylmethyl-[4-(trans-4-(trans-4-heptylcyclohexyl)-cyclohexylethyl)-2,3-difluorphenyl]-ether
trans-4-Pentylcyclohexylmethyl-[4-(trans-4-(trans-4-heptylcyclohexyl)-cyclohexylethyl)-2,3-difluorphenyl]-ether
trans-4-Hexylcyclohexylmethyl-[4-(trans-4-(trans-4-heptylcyclohexyl)-cyclohexylethyl)-2,3-difluorphenyl]-ether

## Beispiel 3

Eine Lösung von 0,05 mol 4-(trans-4-(trans-4-n-Propylcyclohexyl)-cyclohexylethyl-2,3-difluorphenol (herstellbar durch Umsetzung von 2,3-Difluoranisol mit äquimolaren Mengen an t-BuOK und BuLi in THF bei -90° und Alkylierung der Kaliumverbindung mit trans-4-(trans-4-n-Propylcyclohexyl)cyclohexylethyljodid in Gegenwart einer äquimolaren Menge von DMPU und anschließende Etherspaltung mit Bromwasserstoffsäure/Eisessig) und 0,05 mol trans-4-n-Pentylcyclohexancarbonsäure in Methylenchlorid (70 ml) werden bei 0 °C in Gegenwart einer katalytischen Menge von 4-N,N'-Dimethylaminopyridin (DMAP) mit 0,05 mol Dicyclohexylcarbodiimid (DCC) gelöst in Methylenchlorid versetzt. Man läßt das Reaktionsgemisch 12 Stunden bei Raumtemperatur rühren, trennt den ausgefallenen Dicyclohexylharnstoff ab und arbeitet wie üblich auf. Man erhält trans-4-Pentylcyclohexancarbonsäure-[4-(trans-4-(trans-4-propylcyclohexyl)-cyclohexylethyl)-2,3-difluorphenyl]-ester.

Analog werden hergestellt:

trans-4-Pentylcyclohexancarbonsäure-[4-(trans-4-(trans-4-butylcyclohexyl)-cyclohexylethyl)-2,3-difluorphenyl]-ester
trans-4-Pentylcyclohexancarbonsäure-[4-(trans-4-(trans-4-ethylcyclohexyl)-cyclohexylethyl)-2,3-difluorphenyl]-ester
trans-4-Pentylcyclohexancarbonsäure-[4-(trans-4-(trans-4-pentylcyclohexyl)-cyclohexylethyl)-2,3-difluorphenyl]-ester
trans-4-Pentylcyclohexancarbonsäure-[4-(trans-4-(trans-4-heptylcyclohexyl)-cyclohexylethyl)-2,3-difluorphenyl]-ester
trans-4-Propylcyclohexancarbonsäure-[4-(trans-4-(trans-4-ethylcyclohexyl)-cyclohexylethyl)-2,3-difluorphenyl]-ester
trans-4-Propylcyclohexancarbonsäure-[4-(trans-4-(trans-4-propylcyclohexyl)-cyclohexylethyl)-2,3-difluorphenyl]-ester
trans-4-Propylcyclohexancarbonsäure-[4-(trans-4-(trans-4-butylcyclohexyl)-cyclohexylethyl)-2,3-difluorphen

yl]-ester
trans-4-Propylcyclohexancarbonsäure-[4-(trans-4-(trans-4-pentylcyclohexyl)-cyclohexylethyl)-2,3-difluorphenyl]-ester
trans-4-Butylcyclohexancarbonsäure-[4-(trans-4-(trans-4-ethylcyclohexyl)-cyclohexylethyl)-2,3-difluorphenyl]-ester
trans-4-Butylcyclohexancarbonsäure-[4-(trans-4-(trans-4-propylcyclohexyl)-cyclohexylethyl)-2,3-difluorphenyl]-ester
trans-4-Butylcyclohexancarbonsäure-[4-(trans-4-(trans-4-butylcyclohexyl)-cyclohexylethyl)-2,3-difluorphenyl]-ester
trans-4-Butylcyclohexancarbonsäure-[4-(trans-4-(trans-4-pentylcyclohexyl)-cyclohexylethyl)-2,3-difluorphenyl]-ester
trans-4-Heptylcyclohexancarbonsäure-[4-(trans-4-(trans-4-ethylcyclohexyl)-cyclohexylethyl)-2,3-difluorphenyl]-ester
trans-4-Heptylcyclohexancarbonsäure-[4-(trans-4-(trans-4-propylcyclohexyl)-cyclohexylethyl)-2,3-difluorphenyl]-ester
trans-4-Heptylcyclohexancarbonsäure-[4-(trans-4-(trans-4-pentylcyclohexyl)-cyclohexylethyl)-2,3-difluorphenyl]-ester
trans-4-Heptylcyclohexancarbonsäure-[4-(trans-4-(trans-4-heptylcyclohexyl)-cyclohexylethyl)-2,3-difluorphenyl]-ester

Beispiel 4

Unter Feuchtigkeitsausschluß und Stickstoffatmosphäre werden zu einer Lösung von 0,2 mol Ethoxy-2,3-difluorbenzol und 0,2 mol TMEDA in 400 ml THF bei -70 °C 0,21 mol einer 1,6n-Lösung von n-BuLi in Hexan getropft. Man rührt 4 Stunden bei -70°und gibt dann 0,2 mol 4-(trans-4-Pentylcyclohexyl)-cyclohexanon zu. Man läßt das Reaktionsgemisch auf Raumtemperatur erwärmen und hydrolysiert dann mit verdünnter HCl und isoliert das Produkt wie üblich. Das Produkt wird in Toluol aufgenommen und in Gegenwart von einer katalytischen Menge p-Toluolsulfonsäure an Wasserabscheider zum Sieden erhitzt. Danach wäscht man die Toluolphase mit Wasser neutral und dampft sie ein. Der Rückstand wird in THF aufgenommen und mit 30 g Pd/C 5 % E 101 RW bei 23 °C und Normaldruck hydriert. Nach dem Entfernen des Katalysators wird das Lösungsmittel abgedampft und das Produkt durch Chromatographie isoliert. Man erhält 2,3-Difluor-4-[trans-4-(trans-4-pentylcyclohexyl)-cyclohexyl]-1-ethoxybenzol mit K 53° $S_B$ 79° N 184,2° I.

Analog werden hergestellt:

2,3-Difluor-4-[trans-4-(trans-4-ethylcyclohexyl)-cyclohexyl]-1-ethoxybenzol
2,3-Difluor-4-[trans-4-(trans-4-propylcyclohexyl)-cyclohexyl]-1-ethoxybenzol
2,3-Difluor-4-[trans-4-(trans-4-butylcyclohexyl)-cyclohexyl]-1-ethoxybenzol
2,3-Difluor-4-[trans-4-(trans-4-heptylcyclohexyl)-cyclohexyl -1-ethoxybenzol
2,3-Difluor-4-[trans-4-(trans-4-ethylcyclohexyl)-cyclohexyl]-1-pentyloxybenzol
2,3-Difluor-4-[trans-4-(trans-4-propylcyclohexyl)-cyclohexyl]-1-pentyloxybenzol
2,3-Difluor-4-[trans-4-(trans-4-butylcyclohexyl)-cyclohexyl]-1-pentyloxybenzol
2,3-Difluor-4-[trans-4-(trans-4-pentylcyclohexyl)-cyclohexyl]-1-pentyloxybenzol
2,3-Difluor-4-[trans-4-(trans-4-ethylcyclohexyl)-cyclohexyl]-1-octyloxybenzol
2,3-Difluor-4-[trans-4-(trans-4-propylcyclohexyl)-cyclohexyl]-1-octyloxybenzol
2,3-Difluor-4-[trans-4-(trans-4-butylcyclohexyl)-cyclohexyl]-1-octyloxybenzol
2,3-Difluor-4-[trans-4-(trans-4-pentylcyclohexyl)-cyclohexyl]-1-octyloxybenzol

Beispiel 5

Analog Beispiel 4 erhält man durch Umsetzung mit 4-Pentylcyclohexanon 2,3-Difluor-4-(trans-4-pentylcyclohexyl)-1-ethoxybenzol.

Analog werden hergestellt:

2,3-Difluor-4-(trans-4-ethylcyclohexyl)-1-ethoxybenzol
2,3-Difluor-4-(trans-4-propylcyclohexyl)-1-ethoxybenzol
2,3-Difluor-4-(trans-4-butylcyclohexyl)-1-ethoxybenzol

2,3-Difluor-4-(trans-4-heptylcyclohexyl)-1-ethoxybenzol
2 3-Difluor-4-(trans-4-ethylcyclohexyl)-1-propoxybenzol
2,3-Difluor-4-(trans-4-propylcyclohexyl)-1-propoxybenzol
2,3-Difluor-4-(trans-4-butylcyclohexyl)-1-propoxybenzol
2,3-Difluor-4-(trans-4-pentylcyclohexyl)-1-propoxybenzol
2,3-Difluor-4-(trans-4-heptylcyclohexyl)-1-propoxybenzol
2,3-Difluor-4-(trans-4-ethylcyclohexyl)-1-pentyloxybenzol
2,3-Difluor-4-(trans-4-propylcyclohexyl)-1-pentyloxybenzol
2,3-Difluor-4-(trans-4-butylcyclohexyl)-1-pentyloxybenzol
2,3-Difluor-4-(trans-4-pentylcyclohexyl)-1-pentyloxybenzol
2,3-Difluor-4-(trans-4-ethylcyclohexyl)-1-octyloxybenzol 2,3-Difluor-4-(trans-4-propylcyclohexyl)-1-octyloxybenzol
2,3-Difluor-4-(trans-4-pentylcyclohexyl)-1-octyloxybenzol

Beispiel 6

Analog Beispiel 3 erhält man durch Umsetzung von 4-(trans-4-pentylcyclohexylethyl)-2,3-difluorphenol mit 4-Octyloxybenzoesäure 4-Octyloxybenzoesäure-[4-(trans-4-pentylcyclohexylethyl)-2,3-difluorphenyl]-ester mit K 53° $S_C$ (21°) N 137° I.

Analog werden hergestellt:

4-Octyloxybenzoesäure-[4-(trans-4-ethylcyclohexylethyl)-2,3-difluorphenyl]-ester
4-Octyloxybenzoesäure-[4-(trans-4-propylcyclohexylethyl)-2,3-difluorphenyl]-ester
4-Octyloxybenzoesäure-[4-(trans-4-butylcyclohexylethyl)-2,3-difluorphenyl]-ester
4-Octyloxybenzoesäure-[4-(trans-4-heptylcyclohexylethyl)-2,3-difluorphenyl]-ester
4-Pentyloxybenzoesäure-[4-(trans-4-ethylcyclohexylethyl)-2,3-difluorphenyl]-ester
4-Pentyloxybenzoesäure-[4-(trans-4-propylcyclohexylethyl)-2,3-difluorphenyl]-ester
4-Pentyloxybenzoesäure-[4-(trans-4-pentylcyclohexylethyl)-2,3-difluorphenyl]-ester
4-Pentyloxybenzoesäure-[4-(trans-4-heptylcyclohexylethyl)-2,3-difluorphenyl]-ester
4-Propyloxybenzoesäure-[4-(trans-4-ethylcyclohexylethyl)-2,3-difluorphenyl]-ester
4-Propyloxybenzoesäure-[4-(trans-4-propylcyclohexylethyl)-2,3-difluorphenyl]-ester
4-Propyloxybenzoesäure-[4-(trans-4-pentylcyclohexylethyl)-2,3-difluorphenyl]-ester
4-Propyloxybenzoesäure-[4-(trans-4-heptylcyclohexylethyl)-2,3-difluorphenyl]-ester
4-Ethoxybenzoesäure-[4-(trans-4-propylcyclohexylethyl)-2,3-difluorphenyl]-ester
4-Ethoxybenzoesäure-[4-(trans-4-butylcyclohexylethyl)-2,3-difluorphenyl]-ester
4-Ethoxybenzoesäure-[4-(trans-4-pentylcyclohexylethyl)-2,3-difluorphenyl]-ester
4-Ethoxybenzoesäure-[4-(trans-4-hexylcyclohexylethyl)-2,3-difluorphenyl]-ester

Beispiel 7

Analog Beispiel 3 erhält man durch Umsetzung von 4-(trans-4-Pentylcyclohexylethyl)-2,3-difluorphenol mit Capronsäure den entsprechenden Capronsäure-[4-(trans-4-pentylcyclohexylethyl)-2,3-difluorphenyl]-ester mit K 45° N (26,4°) I.

Analog werden hergestellt:

Capronsäure-[4-(trans-4-propylcyclohexylethyl)-2,3-difluorphenyl]-ester
Capronsäure-[4-(trans-4-butylcyclohexylethyl)-2,3-difluorphenyl]-ester
Capronsäure-[4-(trans-4-ethylcyclohexylethyl)-2,3-difluorphenyl]-ester
Capronsäure-[4-(trans-4-heptylcyclohexylethyl)-2,3-difluorphenyl]-ester
Propionsäure-[4-(trans-4-ethylcyclohexylethyl)-2,3-difluorphenyl]-ester
Propionsäure-[4-(trans-4-propylcyclohexylethyl)-2,3-difluorphenyl]-ester
Propionsäure-[4-(trans-4-butylcyclohexylethyl)-2,3-difluorphenyl]-ester
Propionsäure-[4-(trans-4-pentylcyclohexylethyl)-2,3-difluorphenyl]-ester
Propionsäure-[4-(trans-4-heptylcyclohexylethyl)-2,3-difluorphenyl]-ester

Beispiel 8

Ein Gemisch aus 0,25 m 1,2-Difluorbenzol, 0,2 m Kaliumtert.-butylat und 200 ml THF wird bei -90° bis -100° unter Rühren mit 130 ml eine 1,6 n Lösung von Butyllithium (BuLi) versetzt. Danach wird bei -85° bis -90° ein Gemisch aus 0,2 m trans-4-Pentylcyclohexylethyliodid, 0,2 m DMPU und 50 ml THF zugegeben.

Nachdem die Zugabe beendet ist, läßt man die Reaktionsmischung auf -40° erwärmen, rührt noch 1 Stunde nach, gibt Wasser zu und arbeitet wie üblich auf.

Das so erhaltene trans-4-Pentylcyclohexylethyl-2,3-difluorbenzol (0,1 m) wird mit 0,1 m TMEDA in 200 ml THF gelöst, und bei -60° bis -70° wird 0,1 m einer 1,6 n Lösung von BuLi in Hexan zugegeben und gerührt. Nach 2 Stunden Reaktionszeit setzt man ~ 10-20 g Trockeneis (zerkleinert) zu. Man läßt noch 1/2 Stunde nachrühren, läßt auf Raumtemperatur kommen, versetzt mit Wasser und arbeitet wie üblich auf.

Anschließend wird mit 4-Pentylphenol in Gegenwart von DMAP und DCC (Dicyclohexylcarbodiimid) in $CH_2Cl_2$ verestert. Nach Aufarbeiten und Reinigung erhält man 4-(trans-4-Pentylcyclohexylethyl)-2,3-difluorbenzoesäure-(4-pentylphenyl)ester mit K 45° $S_A$ 92° N 130,3° I.

Analog werden hergestellt:

4-(trans-4-Ethylcyclohexylethyl)-2,3-difluorbenzoesäure-(4-pentylphenyl)ester
4-(trans-4-propylcyclohexylethyl)-2,3-difluorbenzoesäure-(4-pentylphenyl)ester
4-(trans-4-Butylcyclohexylethyl)-2,3-difluorbenzoesäure-(4-pentylphenyl)ester
4-(trans-4-Propylcyclohexylethyl)-2,3-difluorbenzoesäure-(4-ethylphenyl)ester
4-(trans-4-Butylcyclohexylethyl)-2,3-difluorbenzoesäure-(4-ethylphenyl)ester
4-(trans-4-Pentylcyclohexylethyl)-2,3-difluorbenzoesäure-(4-ethylphenyl)ester
4-(trans-4-Heptylcyclohexylethyl)-2,3-difluorbenzoesäure-(4-ethylphenyl)ester
4-(trans-4-Ethylcyclohexylethyl)-2,3-difluorbenzoesäure-(4-propylphenyl)ester
4-(trans-4-Propylcyclohexylethyl)-2,3-difluorbenzoesäure-(4-propylphenyl)ester
4-(trans-4-Butylcyclohexylethyl)-2,3-difluorbenzoesäure-(4-propylphenyl)ester
4-(trans-4-Pentylcyclohexylethyl)-2,3-difluorbenzoesäure-(4-propylphenyl)ester
4-(trans-4-Hexylcyclohexylethyl)-2,3-difluorbenzoesäure-(4-propylphenyl)ester
4-(trans-4-Heptylcyclohexylethyl)-2,3-difluorbenzoesäure-(4-propylphenyl)ester
4-(trans-4-Octylcyclohexylethyl)-2,3-difluorbenzoesäure-(4-propylphenyl)ester

Beispiel 9

0,1 m trans-4-Pentylcyclohexylethyl-2,3-difluorbenzol (Herstellung siehe Beispiel 8) und 0,1 m TMEDA werden in 200 ml THF bei -60° bis -70° mit 0,105 m einer 1,6 n Lösung von BuLi in Hexan versetzt. Man rührt 3 Stunden und gibt dann 0,10 m Pentylcyclohexanon (in THF) bei -60° bis -70° zu. Man rührt noch 3 Stunden, wobei das Reaktionsgemisch Raumtemperatur erreichen darf. Anschließend wird mit ges. $NH_4Cl$-Lösung hydrolysiert und wie üblich aufgearbeitet. Der Rückstand wird in Toluol gelöst und mit 3 g p-Toluolsulfonsäure 2 Stunden am Wasserabscheider erhitzt. Nach üblicher extraktiver Aufarbeitung erhält man das entsprechende Cyclohexen-Derivat.

Dieses Alken wird durch Umsetzen mit Dichlordicyanobenzochinon (DDQ) aromatisiert. Dazu gibt man zu einem Gemisch aus 0,155 m Cyclohexenverbindung in 600 ml Toluol bei Raumtemperatur 0,35 m DDQ in 300 ml Toluol. Anschließend erhitzt man 3 Stunden zum Sieden, kühlt auf Raumtempertatur ab, saugt vom Hydrochinon ab und wäscht mit Bisulfit, $H_2O$ und ges. NaCl-Lösung. Nach Aufarbeitung und Reinigung durch Chromatographie und/oder Destillation erhält man 4-(trans-4-Pentylcyclohexylethyl)-2,3-difluor-4′-pentylbiphenyl mit K 22° N 106,7° I.

Analog werden hergestellt:

4-(trans-4-Ethylcyclohexylethyl)-2,3-difluor-4′-pentylbiphenyl
4-(trans-4-Propylcyclohexylethyl)-2,3-difluor-4′-pentylbiphenyl
4-(trans-4-Butylcyclohexylethyl)-2,3-difluor-4′-pentylbiphenyl
4-(trans-4-Heptylcyclohexylethyl)-2,3-difluor-4′-pentylbiphenyl
4-(trans-4-Nonylcyclohexylethyl)-2,3-difluor-4′-pentylbiphenyl
4-(trans-4-Ethylcyclohexylethyl)-2,3-difluor-4′-heptylbiphenyl
4-(trans-4-Propylcyclohexylethyl)-2,3-difluor-4′-heptylbiphenyl
4-(trans-4-Pentylcyclohexylethyl)-2,3-difluor-4′-heptylbiphenyl

4-(trans-4-Heptylcyclohexylethyl)-2,3-difluor-4′-heptylbiphenyl
4-(trans-4-Ethylcyclohexylethyl)-2,3-difluor-4′-propylbiphenyl
4-(trans-4-Propylcyclohexylethyl)-2,3-difluor-4′-propylbiphenyl
4-(trans-4-Butylcyclohexylethyl)-2,3-difluor-4′-propylbiphenyl
4-(trans-4-Pentylcyclohexylethyl)-2,3-difluor-4′-propylbiphenyl
4-(trans-4-Hexylcyclohexylethyl)-2,3-difluor-4′-propylbiphenyl
4-(trans-4-Ethylcyclohexylethyl)-2,3-difluor-4′-ethylbiphenyl
4-(trans-4-Propylcyclohexylethyl)-2,3-difluor-4′-ethylbiphenyl
4-(trans-4-Pentylcyclohexylethyl)-2,3-difluor-4′-ethylbiphenyl
4-(trans-4-Heptylcyclohexylethyl)-2,3-difluor-4′-ethylbiphenyl
4-(trans-4-Octylcyclohexylethyl)-2,3-difluor-4′-ethylbiphenyl

Das folgende Beispiel betrifft eine erfindungsgemäße flüssigkristalline phase:

Beispiel A:

Eine nematische flüssigkristalline Phase bestehend aus
13 % p-trans-4-Propylcyclohexyl-benzonitril,
20 % trans-1-p-Methoxyphenyl-4-propylcyclohexan,
13 % trans-1-p-Butoxyphenyl-4-propylcyclohexan,
17 % 2,3-Difluor-4-(trans-4-pentylcyclohexyl)-1-ethoxybenzol
3 % trans-4-(trans-4-Propylcyclohexyl)-cyclohexancarbonsäure-(trans-4-propylcyclohexylester),
3 % trans-4-(trans-4-Propylcyclohexyl)-cyclohexancarbonsäure-(trans-4-pentylcyclohexylester),
3 % trans-4-(trans-4-Butylcyclohexyl)-cyclohexancarbonsäure-(trans-4-pentylcyclohexylester),
3 % trans-4-(trans-4-Butylcyclohexyl)-cyclohexancarbonsäure-(trans-4-propylcyclohexylester),
5 % 4,4′-(trans-4-Propylcyclohexyl)-2-fluorbiphenyl,
4 % 4,4′-(trans-4-Propylcyclohexyl)-biphenyl,
4 % 4,4′-(trans-4-Pentylcyclohexyl)-2-fluorbiphenyl,
6 % 2,3-Difluor-4-[trans-4-(trans-4-pentylcyclohexyl)-cyclohexyl]-1-ethoxybenzol und
6 % 4-(trans-4-Pentylcyclohexyl)-4′-(trans-4-propylcyclohexyl)-biphenyl
zeichnet sich durch einen breiten nematischen Phasenbereich und eine niedrige Doppelbrechung aus.

## Patentansprüche

1. Difluorbenzolderivate der Formel I,

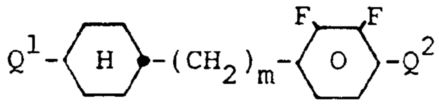

I

worin

$Q^1$ und $Q^2$ jeweils unabhängig voneinander R oder R-$A^1$-$A^2$-Z bedeuten, wobei

R jeweils unsubstituiertes, einfach durch Cyan oder mindestens einfach durch Fluor oder Chlor substituiertes Alkyl mit 1-15 C-Atomen oder Alkenyl mit 3-15 C-Atomen, wobei in diesen Resten auch eine $CH_2$-Gruppe durch -O-, -CO-, -O-CO-, -CO-O-oder -O-CO-O- ersetzt sein kann,

$A^1$ und $A^2$ jeweils unabhängig voneinander trans-1,4-Cyclohexylen, worin auch eine oder zwei nicht benachbarte $CH_2$-Gruppen durch -O-ersetzt sein können, oder unsubstituiertes oder ein oder zweifach durch Fluor substituiertes 1,4-Phenylen, worin auch eine oder zwei CH-Gruppen durch N ersetzt sein können; eine der beiden Gruppen $A^1$ und $A^2$ auch eine Einfachbindung,

Z -CO-O-, -O-CO-, -$CH_2$O-, -O$CH_2$-, -$CH_2CH_2$-oder eine Einfachbindung bedeutet, und

m 0 oder 2 ist,

mit der Maßgabe, daß im Falle

(a) $Q^1$ = Alkyl oder Alkoxy und

$Q^2$ = Alkyl, Alkoxy, 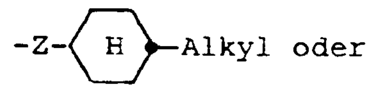 oder

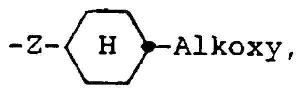, oder

(b) $Q^1$ = 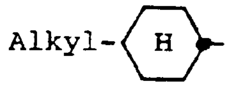 oder 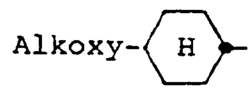 und

$Q^2$ = Alkyl oder Alkoxy

m = 0 bedeutet.

2. Verfahren zur Herstellung von Difluorbenzolderivaten nach Anspruch 1, dadurch gekennzeichnet, daß man eine Verbindung, die an Stelle von H-Atomen eine oder mehrere reduzierbare Gruppen- und/oder C-C-Bindungen enthält, mit einem reduzierenden Mittel behandelt,
oder daß man zur Herstellung von Estern (worin Z -CO-O- oder -O-CO- bedeutet und/oder in R mindestens eine $CH_2$-Gruppe durch -O-CO-, -CO-O- oder -O-COO-ersetzt ist) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Ethern (worin Z $-CH_2O-$ oder $-OCH_2-$ bedeutet und/oder in R mindestens eine $CH_2$-Gruppe durch -O- ersetzt ist) eine entsprechende Hydroxyverbindung verethert.

3. Verwendung der Derivate nach Anspruch 1 als Komponenten flüssigkristalliner Phasen.

4. Flüssigkristalline Phase mit mindestens zwei flüssigkristallinen Komponenten, dadurch gekennzeichnet, daß mindestens eine Komponente ein Derivat nach Anspruch 1 ist.

5. Elektrooptisches Anzeigeelement, dadurch gekennzeichnet, daß es als Dielektrikum eine Phase nach Anspruch 4 enthält.

## Claims

1. Difluorobenzene derivatives of the formula I,

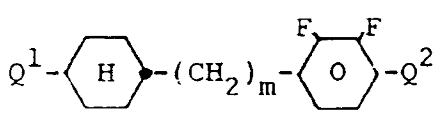 I

wherein

$Q^1$ and $Q^2$ each independently of one another are R or $R-A^1-A^2-Z$,

R being alkyl having 1-15 C atoms or alkenyl having 3-15 C atoms, each of which is unsubstituted or is monosubstituted by cyano or at least monosubstituted by fluorine or chlorine, it also being possible for a $CH_2$ group in these radicals to be replaced by -O-, -CO-, -O-CO-, -CO-O- or -O-CO-O,

$A^1$ and $A^2$ each independently of one another being trans-1,4-cyclohexylene, in which one or two non-adjacent $CH_2$ groups can also be replaced by -O-, or 1,4-phenylene which is unsubstituted or mono- or di-substituted by fluorine and in which one or two CH groups can also be replaced by N, and one of the two groups $A^1$ and $A^2$ also being a single bond, and

Z being -CO-O-, -O-CO-, $-CH_2O-$,$-OCH_2-$, $-CH_2CH_2-$or a single bond, and

m is 0 or 2,

with the proviso that, in the case of

(a) $Q^1$ = alkyl or alkoxy and

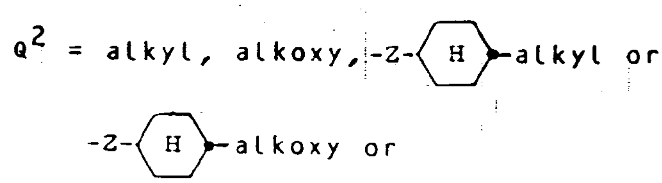

(b) 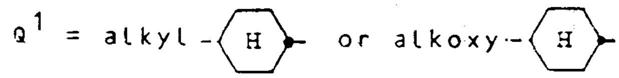 and

$Q^2$ = alkyl or alkoxy

m = 0.

2. Process for the preparation of difluorobenzene derivatives according to Claim 1, characterized in that a compound which, in place of H atoms, contains one or more reducible groups and/or C-C bonds, is treated with a reducing agent, or
that, for the preparation of esters (in which Z is -CO-O-or -O-CO- and/or at least one $CH_2$ group in R is replaced by -O-CO-, -CO-O- or -O-COO-) a corresponding carboxylic acid or a reactive derivative thereof is reacted with a corresponding alcohol or a reactive derivative thereof, or
that, for the preparation of ethers (in which Z is $-CH_2O-$or $-OCH_2-$ and/or at least one $CH_2$ group in R is replaced by -O-), a corresponding hydroxy compound is etherified.

3. Use of the derivatives according to Claim 1 as components of liquid-crystalline phases.

4. liquid-crystalline phase with at least two liquid-crystalline components, characterized in that at least one component is a derivative according to Claim 1.

5. Electro-optical display element, characterized in that it contains a phase according to Claim 4 as the dielectric.

**Revendications**

1. Dérivés de difluorobenzène, de formule I

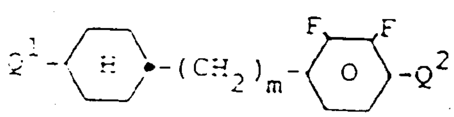

I

dans laquelle

$Q^1$ et $Q^2$ représentent chacun, indépendamment l'un de l'autre, R ou R-$A^1$-$A^2$-Z pour lesquels

R représente dans chaque cas un groupe alkyle en C 1-C 15 ou alcényle en C 3-C 15 non substitué, monosubstitué par un groupe cyano ou substitué au moins une fois par le fluor ou le chlore, et dans lesquels un groupe $-CH_2-$peut également être remplacé par -O-, -CO-, -O-CO-, -CO-O-ou -O-CO-O-,

$A^1$ et $A^2$ représentent chacun, indépendamment l'un de l'autre, un groupe trans-1,4-cyclohexylène dans lequel un ou deux groupes $-CH_2-$ non voisins peuvent être remplacés par -O-, ou un groupe 1,4-phénylène non substitué ou substitué une fois ou deux fois par le fluor et dans lequel également un ou deux groupes -CH- peuvent être remplacés par N ; l'un des deux symboles $A^1$ et $A^2$ pouvant également représenter une liaison simple,

Z représente -CO-O-, -O-CO-, $-CH_2O-$, $-OCH_2-$, $-CH_2CH_2-$ ou une liaison simple, et

m est égal à 0 ou 2,

sous réserve que

(a) lorsque $Q^1$ = alkyle ou alcoxy et
$Q^2$ = alkyle, alcoxy, -Z-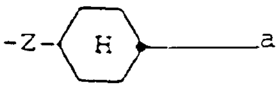alkyl ou

-Z-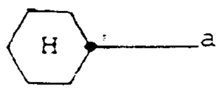alcoxy, ou bien

(b) $Q^1$ = alkyl-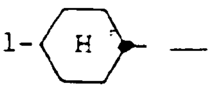 ou alcoxy-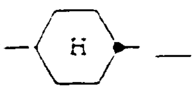 et

$Q^2$ = alkyle ou alcoxy,

m est égal à 0.

2. Procédé de préparation des dérivés de difluorobenzène de la revendication 1, caractérisé en ce que l'on traite par un agent réducteur un composé qui, à la place d'atomes d'hydrogène, contient un ou plusieurs groupes et/ou une ou plusieurs liaisons C-C réductibles,
ou bien, pour la préparation des esters (dans lesquels Z représente -CO-O- ou -O-CO- et/ou, dans R, au moins un groupe $-CH_2-$ est remplacé par -O-CO-, -CO-O- ou -O-COO-) on fait réagir un acide carboxylique correspondant ou l'un de ses dérivés réactifs avec un alcool correspondant ou l'un de ses dérivés réactifs,
ou bien, pour la préparation des éthers (dans lesquels Z représente $-CH_2O-$ ou $-OCH_2-$ et/ou dans R, au moins un groupe $CH_2$ est remplacé par -O-), on éthérifie un dérivé hydroxylé correspondant.

3. Utilisation des dérivés de la revendication 1 en tant que composants de phases à cristaux liquides.

4. Phase à cristaux liquides à au moins deux composants à cristaux liquides, caractérisée en ce que l'un au moins des composants est un dérivé selon la revendication 1.

5. Elément d'affichage électro-optique, caractérisé en ce qu'il contient en tant que diélectrique une phase selon la revendicatien 4.